# EUROPEAN PATENT APPLICATION

(11) **EP 3 207 870 A1**
(43) Date of publication of application: **23.08.2017**
(21) Application number: 16156000.8
(22) Date of filing: 16.02.2016
(51) Int. Cl.: A61B 5/145

(54) **BODY-MOUNTABLE DEVICE, MEDICAL SENSOR ASSEMBLY AND METHOD OF USE**

(71) Applicant: Roche Diabetes Care GmbH, 68305 Mannheim (DE); F. Hoffmann-La Roche AG, 4070 Basel (CH)
(72) Inventor: Harttig, Herbert, D-67434 Neustadt (DE); Kube, Oliver, D-67549 Worms (DE)
(74) Representative: Pfiz, Thomas

(57) **Abstract**

The invention concerns a body-mountable device (12) for a medical sensor assembly (10) and a method of using the same, wherein the device (12) comprises a patch (20) configured for adhesive attachment to a body (16) of a user and a mounting unit (24) arranged on the patch (20) for connecting a disposable transcutaneous sensor (14) to a reusable electronics unit (18). In order to facilitate sterile handling, the mounting unit (24) comprises an electronics compartment (26) which is closed by a sealed cover (28) for fully encasing an internal space (44) adapted for receiving the electronics unit (18), wherein the cover (28) is operable for insertion or removal of the electronics unit (18).

## Description

The invention concerns a body-mountable device for a medical sensor assembly, in particular for continuous analyte monitoring, such as continuous glucose or lactate monitoring, comprising a patch configured for adhesive attachment to a body of a patient and a mounting unit arranged on the patch for connecting a disposable transcutaneous sensor to a reusable electronics unit. The invention further concerns a medical sensor assembly including such a body-mount and a method of using same.

Monitoring of certain analytes, specifically glucose or lactate in body fluids like blood or interstitial fluid may be performed by using electrochemical sensors. In addition to intermittent measurement, in which a user takes a sample by lancing the skin of a boy part, continuous glucose monitoring (CGM) systems are established specifically in clinical practice for controlling a diabetes state. Here, the sensor is partially implanted with its electrode portion in the interstitial tissue for a longer period of at least several days. The sensor is generally applied using an insertion instrument, which is then removed, while a contact portion of the sensor outside the body remains connected to an electronics unit which amplifies the weak analogue measuring signal for further processing.

In this context, it is known from US2012/0197222 to provide in vivo analyte sensors fully integrated with on-body electronics which may include a glucose sensor, electronics, battery and antenna encased (except for the sensor portion that is for in vivo positioning) in a waterproof housing that includes or is attachable to an adhesive pad. However, such an arrangement requires the electronics unit to be disposed after the limited lifetime of the sensor. Alternatively, it has been practiced to clean and disinfect the electronics unit, though the increased time and effort may hardly be accepted particularly when clinical personnel are involved.

On this basis the object of the invention is to further improve the known devices, systems and methods and to provide a design which allows cost-effective and easy to handle use and while reducing the risk of infectious contamination and mismeasurement.

The combination of features stated in the independent claims is proposed to achieve this object. Advantageous embodiments and further developments of the invention are derived from the dependent claims.

The invention is based on the idea of providing the electronics unit completely shielded against the sensor. Accordingly, it is proposed that the mounting unit comprises an electronics compartment which is closed by a sealing cover for fully encasing an internal space adapted for receiving the electronics unit, wherein the cover can be opened for insertion or removal of the electronics unit. In this configuration, it is possible to package the electronics in a germ-and fluid-tight manner, while significantly reducing the effort for reuse. Cleaning and disinfection may even be avoided as the electronics is fully encased and not exposed to any body fluid. Moreover, a contamination of contact part of the electronics with body fluid can be avoided, thereby reducing the risk of incorrect measurements.

Advantageously, the electronics compartment is formed integrally with the mounting unit preferably as a molded part which may be fabricated by injection molding. In this way, manufacturing costs may be reduced, and handling of separated parts can be avoided. Thus, the body-mount may easily be disposable as a single use article, while the electronics can be reused.

Preferably, the electronics compartment is formed by a shell or vessel surrounding an opening, and wherein a rim of the shell or vessel forms a seal seat for a rim of the cover. In order to improve the tightness, additional sealing elements such as an elastomer seal or a past-like sealant can be easily integrated.

For further handling improvement it is advantageous when the cover is connected by an integral hinge-joint to the mounting unit, specifically to the electronics compartment.

It is also preferred that the internal space of the electronics compartment is adapted for a form-locked accommodation of the electronics unit in a defined orientation. Thus, a misalignment can be avoided.

Another handling improvement provides that the cover is operable by means for a lever which is attached to the mounting unit or to the cover.

In this connection it is further advantageous when the cover on its side facing the internal space is provided with a hold-down element to bias the electronics unit against electrical contacts, thereby maintaining a reliable electric connection.

A particular embodiment further comprises an electrical through-connection between the internal space and the exterior of the electronics compartment that is provided through a wall of the electronics compartment for a galvanic connection of the sensor to the electronics unit. In this way, the functionality of the electronics can be maintained in a germ-shielded space.

For further design improvement it is advantageous when the electrical through-connection comprises contact pads which are arranged on both sides of the wall and which consist of one of metal, carbon and electrically conductive polymer.

According to a preferred implementation, the mounting unit comprises a pressure element configured to pressure the sensor against electrical contacts on the electronics compartment, thereby avoiding contact faults.

Advantageously, the mounting unit further comprises a platform for connecting a proximal part of the sensor, wherein the electronics compartment is pivot-mounted on the platform for providing access to the implantation site.

The invention further relates to a medical sensor assembly, in particular for continuous analyte monitoring, comprising a body-mountable device, a disposable transcutaneous sensor and a reusable electronics unit connected to the sensor for receiving a sensor signal which is indicative for an analyte in a body fluid, wherein the electronics unit is fully encased in an electronics compartment which is closed by a sealed cover being operable to remove the electronics unit.

Preferably, the sensor is arranged completely on the exterior of the electronics compartment and is connected to the electronics unit by means of an electrical through-connection.

Another aspect of the invention is directed to a method for application of a body-mountable device in a medical sensor assembly, comprising
- providing a body-mountable device having an adhesive patch and a mounting unit arranged thereon for connecting a disposable transcutaneous sensor to a reusable electronics unit, wherein the mounting unit comprises an electronics compartment which is closeable by a sealed cover,
- sterilizing the body-mountable device in a sterile-tight package in a place of manufacture,
- opening the sterile-tight package in a place of use for removal of the body-mountable device,
- inserting an electronics unit into the electronics compartment and closing the electronics compartment by means of the cover,
- attaching the patch and the mounting unit arranged thereon to a body of a user.

In this connection, it is also advantageous when providing access to a sensor implantation site on the body through a pivoting motion of the electronics compartment, while the electronics compartment is pivot-mounted on a platform of the mounting unit.

In the following, the invention is further elucidated on the basis of an embodiment example shown schematically in the drawings, where
- Fig. 1: is a cross-sectional view of a medical sensor assembly comprising a body-mountable device;
- Fig. 2: is a plan view of an electro-chemical sensor;
- Fig. 3: is an enlarged detail of Fig. 1;
- Fig. 4 and 5: is a cross-sectional view and a top view of a different embodiment of a medical sensor assembly;
- Fig. 6: to 9 show subsequent steps of application of the body-mountable device in the embodiment of Fig. 1.

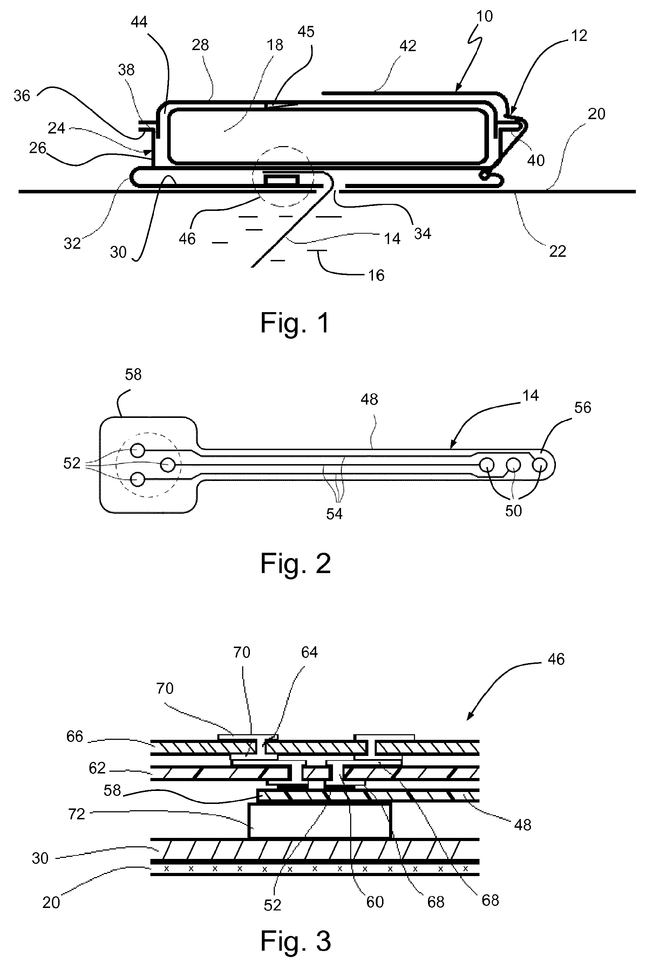

In Fig. 1, an exemplary embodiment of a medical sensor assembly 10 for continuous glucose monitoring is shown. The assembly 10 comprises a body-mount 12, an electro-chemical glucose sensor 14 inserted into a body part 16 of a patient and an electronics unit 18.

The body-mount 12 includes a patch 20 for adhesive attachment on the skin 22 of the body part 16 and a mounting unit 24 for a sterile encasing and galvanic connection of the electronics unit 18 to the sensor 14. For this purpose, the mounting unit 24 comprises an electronics compartment 26 which is closed by a sealing cover 28. As a base of the mounting unit 24, a platform 30 is glued to the upper surface of the patch 20 facing away from the skin 22. In order to permit skin insertion of the sensor 14, the electronics compartment 26 is pivot-mounted on the platform 30 by means of an integral articulation 32. In the implanted state, the sensor 14 reaches through a hole 34 in patch 20 and platform 30.

The electronics compartment 26 may be formed integrally with the platform 30 preferably as a molded plastic part. It may be construed as a vessel 36 which surrounds an opening and forms a seal seat 38 for the cover 28. For simplification of handling, the cover 28 may be connected by a hinge-joint 40 to the electronics compartment 26 and may be operated and/or secured by means of at least one lever 42. On its side facing the internal space 44 of the electronics compartment 26, the cover 28 may be provided with a hold-down element 45 to bias the electronics unit 18 against an electrical contact arrangement 46, as further described below with reference to Fig. 3.

Fig. 2 shows the electro-chemical sensor 14 in a state prior to application. A flexible substrate 48 is provided as a carrier for electrodes 50 and contact pads 52 which are interconnected by electrical traces 54. The electrodes 50 are arranged on a distal end of an elongated shaft 56 which can be inserted transcutaneously into the body 16 by means of an insertion aid (not shown), as known *per se* to the skilled person. The contact pads 52 are provided on a widened contact portion 58 of the substrate 48 for a detachable electrical interconnection with the electronics unit 18.

The electrodes 50 are adapted for performing an electrochemical detection reaction for detecting an analyte (e.g. glucose) in body fluid (e.g. interstitial fluid or blood). For this purpose, a test chemical may be deposited on a working electrode and a counter electrode may be adapted for balancing a current flow required by the detection reaction at the working electrode. Additionally, a reference electrode may provide a stable electrode potential. A current as low as a few nanoamperes may result as a measurement signal, which has to be conducted to the electronics unit 18 for signal processing.

As shown in more detail in Fig. 3, the contact arrangement 46 provides a first electrical through-connection 60 through a base wall 62 of the electronics compartment 26 and a second electrical through-connection 64 through a housing wall 66 of the electronics unit 18. The through-connections 60, 64 may be provided as a so called via (vertical interconnect access) as known in printed circuit board design. These vias may end in contact areas 68, 70 in corresponding positions on both sides of the walls 62, 66. In this context, corresponding positions means that the size and arrangement should be designed in such a way that an effectual contact security is achieved even under given positional tolerances. The contact areas 68, 70 may consist of metal, carbon or electrically conductive polymers. In order to provide sufficient contact force, a pressure element 72 is arranged on the platform 30 opposite to the contact pads 52 on the contact portion 58 of the sensor substrate 48.

The electronics unit 18 comprises an amplifier for amplification of the analogue current signal and a transmitter for transmitting measurement data to a receiver outside the sensor assembly preferably by a wireless connection. Further components of the electronics unit 18 may include a battery or accumulator and a memory for intermediate storage of measurement data. The interior of the electronics unit 18 is not detailed in the drawings.

As such, the electronics unit 18 has a lasting worth and may be designed for possible re-use, whereas the lifetime of the remaining components of the sensor assembly 10 is relatively short, which makes them disposable. Specifically, the sensor 14 itself has a limited running life in the implanted state. Also, the patch 20 must be renewed after a given period of e.g. one, two or up to four weeks of attachment to the skin.

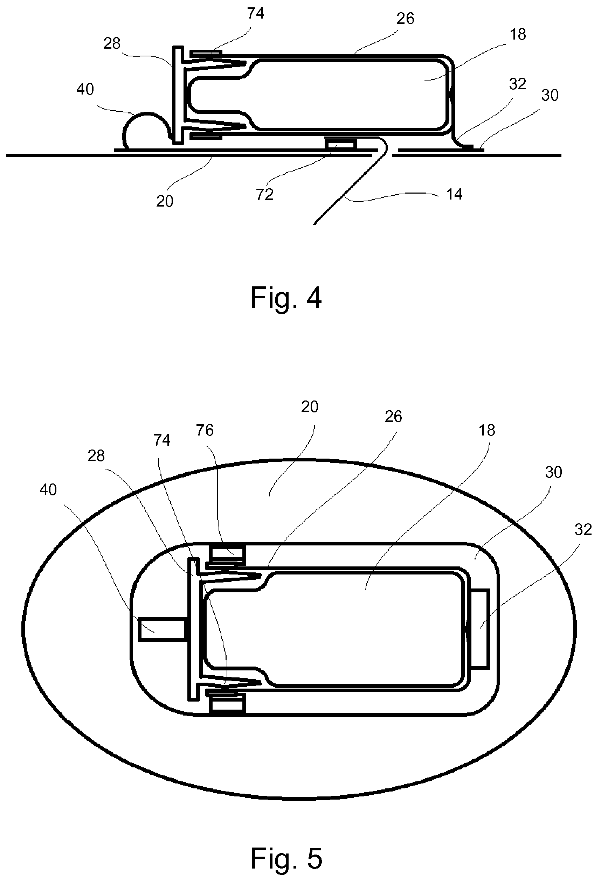

Figs. 4 and 5 show a second embodiment, where similar parts are provided with similar reference numerals, as explained above. As a difference, it can be seen that the electronics unit 18 has a more elongated shape with a narrowed neck and an approximately elliptical cross-section, in contrast to the disk shape of the electronics unit 18 shown in Fig. 1. The cover 28 is formed as a plugging lid which opens to the side and has a sealing rim extension 74. Thus, the electronics unit 18 and the electronics compartment 26 including the cover 28 have a corresponding geometry to allow a form-locked accommodation in only a well-defined orientation. Conveniently, the electronics compartment 26 is detachably locked by snap elements 76 in place on the contact portion of the sensor 14. However, a combination with a locking lever 42 as shown in Fig. 1 also may be feasible.

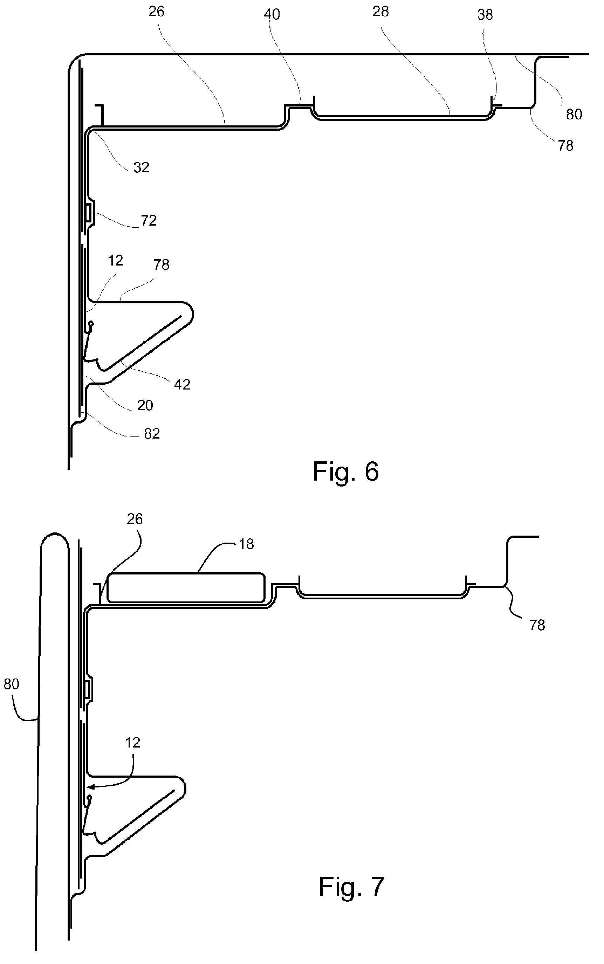

The use of the disposable body-mount 12 in combination with the re-usable electronics unit 18 is illustrated in simplified cross-sectional views of Fig. 6 to 9. At the outset, it should be noted that the reuse of an electronics unit in a conventional assembly requires thorough cleaning and disinfection, as it could come in contact with body fluid during use. Specifically, this applies during use for various patients in a professional clinical environment.

As depicted in Fig. 6, the disposable body-mount 12 according to the invention may be provided in a sterile-tight blister package 78 in a place of manufacture. There, the body-mount 12 including the electronics compartment 26 and the cover 28 may be sterilized using a gas sterilization technique without impairing the adhesiveness of the pad 20, which may be covered by a liner 82. In a certain embodiment, ethylene dioxide as sterilizing gas is introduced and released through a gas-permeable membrane 80 sealing the blister package 78. This also allows for storage during a given shelf-life without interfering sterility within the package 78.

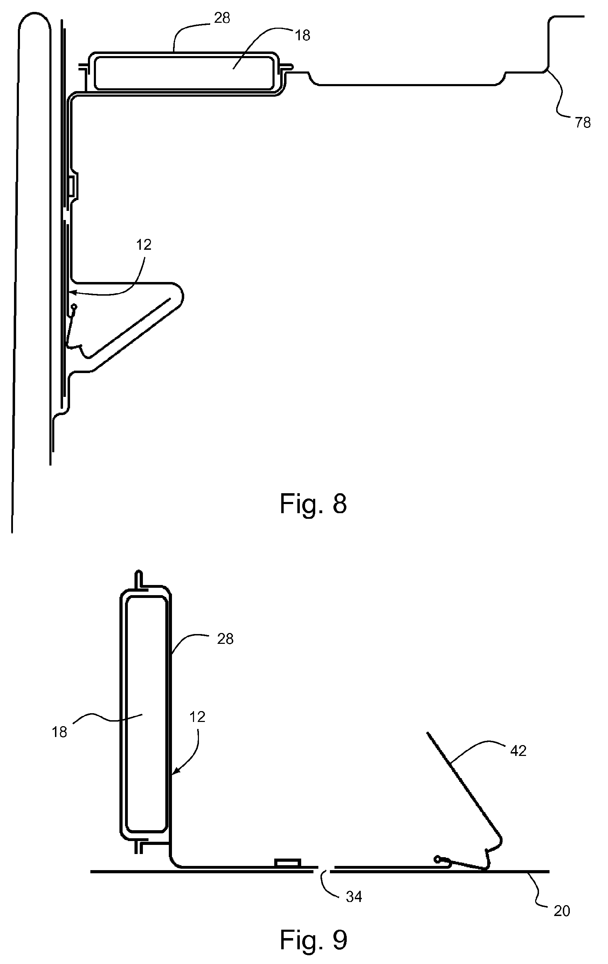

In a next step, as illustrated in Fig. 7, the blister package 78 may be opened in a place of use by removing the membrane 80. Thereafter, the electronics unit 18 may be inserted into the compartment 26. Beforehand, the housing of the electronics unit 18 may be cleaned and disinfected, which is aided by providing a smooth and seamless surface structure. However, as the electronics unit 18 can be fully encased by closing the cover, as shown in Fig. 8, disinfection may be even obsolete.

Then, as can be seen in Fig. 9, the patch 20 and the mounting unit 12 arranged thereon can be adhered onto the skin of a body part. In order to allow implantation of the sensor 14, the electronics compartment 28 may be pivoted by an angle e.g. between 90° and 180° around the articulation or hinge 32. Thereby, the opening 334 is kept free for applying an inserter in order to implant the sensor 14. In a final step, the electronics compartment 28 is turned back and kept in place by means of the locking lever 42, as shown in Fig. 1.

## Claims

1. Body-mountable device for a medical sensor assembly (10), in particular for continuous analyte monitoring, comprising a patch (20) configured for adhesive attachment to a body of a user and a mounting unit (24) arranged on the patch (20) for connecting a disposable transcutaneous sensor (14) to a reusable electronics unit (18), **characterized in that** the mounting unit (24) comprises an electronics compartment (26) which is closed by a sealed cover (28) for fully encasing an internal space (44) adapted for receiving the electronics unit (18), wherein the cover (28) is operable for insertion or removal of the electronics unit (18).

2. The device of claim 1, wherein the electronics compartment (26) is formed integrally with the mounting unit (24) preferably as a molded part.

3. The device of claim 1 or 2, wherein the electronics compartment (26) is formed by a vessel (36) surrounding an opening, and wherein a rim of the vessel (36) forms a seal seat (38) for a rim of the cover (28).

4. The device according to any of claims 1 to 3, wherein the cover (28) is connected by an integral hinge-joint (40) to the mounting unit (24), specifically to the electronics compartment (26).

5. The device according to any of claims 1 to 4, wherein the internal space (44) of the electronics compartment (26) is adapted for a form-locked accommodation of the electronics unit (18) in a defined orientation.

6. The device according to any of claims 1 to 5, wherein the cover (28) is operable by means of a lever (42) which is attached to the mounting unit (24) or to the cover (28).

7. The device according to any of claims 1 to 6, wherein the cover (28) on its side facing the internal space (44) is provided with a hold-down element (45) to bias the electronics unit (18) against electrical contacts (68).

8. The device according to any of claims 1 to 7, further comprising an electrical through-connection (60) between the internal space (44) and the exterior of the electronics compartment (26) that is provided through a wall (62) of the electronics compartment (26) for a galvanic connection of the sensor (14) to the electronics unit (18).

9. The device according to claim 8, wherein the electrical through-connection (60) comprises contact pads (68) which are arranged on both sides of the wall (62) and which consist of at least one of metal, carbon and electrically conductive polymer.

10. The device according to any of claims 1 to 9, wherein the mounting unit (24) comprises a pressure element (72) configured to pressure the sensor (14) against electrical contacts (68).

11. The device according to any of claims 1 to 10, wherein the mounting unit (24) further comprises a platform (30) for connecting a proximal part (58) of the sensor (14), and wherein the electronics compartment (26) is pivot-mounted on the platform (30).

12. Medical sensor assembly, in particular for continuous analyte monitoring, comprising a body-mountable device (12) according to any of the preceding claims, and further comprising a disposable transcutaneous sensor (14) and a reusable electronics unit (18) connected to the sensor (14) for receiving a sensor signal which is indicative for an analyte in a body fluid, **characterized in that** the electronics unit (18) is fully encased in an electronics compartment (26) which is closed by a sealed cover (28) being operable for removing of the electronics unit (18).

13. The medical sensor assembly of claim 11, wherein the sensor (14) is arranged completely on the exterior of the electronics compartment (26) and is connected to the electronics unit (18) by means of an electrical through-connection (60,64).

14. Method for application of a body-mountable device (12) in a medical sensor assembly (10), comprising
- providing a body-mountable device (12) having an adhesive patch (20) and a mounting unit (24) arranged thereon for connecting a disposable transcutaneous sensor (14) to a reusable electronics unit (18), wherein the mounting unit (24) comprises an electronics compartment (26) which is closeable by a sealed cover (28),
- sterilizing the body-mountable device (12) in a sterile-tight package (78) in a place of manufacture,
- opening the sterile-tight package (78) in a place of use for removal of the body-mountable device (12),
- inserting an electronics unit (18) into the electronics compartment (26) and closing the electronics compartment (26) by means of the cover (28),
- attaching the patch (20) and the mounting unit (24) arranged thereon to a body of a user.

15. The method of claim 14, further comprising providing access to a sensor implantation site on the body through a pivoting motion of the electronics compartment (26), wherein the electronics compartment (26) is pivot-mounted on a platform (30) of the mounting unit (24).
